# EUROPEAN PATENT APPLICATION

(11) **EP 3 733 210 A1**
(43) Date of publication of application: **04.11.2020**
(21) Application number: 18894850.9
(22) Date of filing: 28.12.2018
(51) Int. Cl.: A61K 48/00, A61K 47/69, A61P 35/00

(54) **CELL SHEET FOR GENE DELIVERY**

(30) Priority: 29.12.2017 KR 20170183653
(71) Applicant: Genemedicine Co., Ltd., Seoul 04763 (KR)
(72) Inventor: YUN, Chae Ok, Seoul 06583 (KR); KIM, Tae Geuk, Seoul 02791 (KR)
(74) Representative: Plasseraud IP
(86) International application number: PCT/KR2018/016869
(87) International publication number: WO 2019/132594

(57) **Abstract**

The present invention relates to a cell sheet for gene delivery. Unlike conventional cell sheets for tissue regeneration, a cell sheet according to the present invention can be used as a local gene delivery system. Particularly when a virus is used as a gene delivery system, the virus can be proliferated within the cell sheet and acts topically within a therapeutic region. Thus, the cell sheet is superior in the prevention or treatment of cancer, the prevention of cancer recurrence or cancer metastasis, particularly the treatment of multifocal tumor even though the virus dose is remarkably lowered compared to the systemic administration or intratumoral injection of the virus.

## Description

### [Technical Field]

The present invention relates to a cell sheet for gene delivery.

### [Background Art]

Despite the rapid development of cancer therapeutics, cancer is still one of the diseases with high death rates worldwide. The main cancer treatment methods which have been conventionally used in clinics include surgeries, radiation therapy, anticancer drug treatment, a combination thereof, which are for removing as many cancer cells from a patient as possible. However, these treatments are for only relatively early stage cancer and show therapeutic effects only when cancer cells are completely removed without metastasis.

Particularly, hepatocellular carcinoma (HCC) is known as the fifth most common cancer and the third leading cause of cancer-related death worldwide. Chronic liver diseases, such as hepatitis B and hepatitis C viral infections, and cirrhosis caused thereby account for 80 to 90% of all liver cancer cases. One of the key characteristics of HCC is that it often simultaneously develops multiple tumor lesions (multifocal/multicentric/intrahepatic metastases) and such multifocal hepatocytes contribute to a high recurrence rate, a drug resistant property, and morbidity. Importantly, a long-term cohort study showed a strong positive correlation between hepatic cirrhosis and multiple/multifocal HCC. Chronic viral infection is closely related to repetitive hepatocellular necrosis, followed by regeneration. Such an accelerated cell cycle may be associated with the accumulation of genetic errors in the liver, resulting in tumors in many liver sites, and patients with such genetic errors may be categorized as having multifocal HCC.

A current standard therapeutic method for HCC includes selective internal radiation therapy and systemic therapy using a chemotherapeutic agent. However, these treatment modalities lacking cancer specificity are highly toxic to the liver, leading to a serious problem in the majority of HCC patients who exhibit liver dysfunction, and thus it is difficult to administer a sufficient dose of drugs to eradicate the tumors. Due to the above-described reasons, surgical resection remains a preferential therapy. However, most HCC patients (< 70%) are ineligible for resection due to various reasons such as multiple sclerosis and hepatic cirrhosis [El-Serag, H.B., et al., Diagnosis and treatment of hepatocellular carcinoma. Gastroenterology, 2008. 134(6): p. 1752-63; Belghiti, J. and R. Kianmanesh, Surgical treatment of hepatocellular carcinoma. HPB (Oxford), 2005. 7(1): p. 42-9; Ziser, A., et al., Morbidity and mortality in cirrhotic patients undergoing anesthesia and surgery. Anesthesiology, 1999. 90(1): p. 42-53].

Even with curative resection, a high recurrence rate in patients remains a significant challenge for HCC therapy.

Oncolytic virotherapy, which demonstrated potent and cancer-specific cell killing efficacy in various clinical trials, could be a promising candidate to overcome off-target cytotoxicity associated with small molecule chemotherapy.Among several oncolytic vectors, an adenovirus (Ad) has several beneficial features, such as no risk of insertional mutagenesis, facile production in high-titer and a high transgene expression level, that makes it more favorable toward cancer gene therapy. Despite promising preclinical results, several hurdles, such as inadequate delivery and insufficient levels of therapeutic gene transfer or viral replication, should be overcome to elicit optical antitumor efficacy in clinical trials. Importantly, intratumoral inoculation of virions, which remains a preferable administration route in clinical trials due to safety concerns and limited efficacy of systemically administered virions, may not be feasible in case of multifocal tumors. Currently, there are lack of effective and standardized protocols to treat several tumors simultaneously with tumor-killing viruses. Although systemic administration may circumvent these limitations with other standard therapeutics, the highly immunogenic nature of oncolytic viruses and high prevalence of pre-existing immunity against Ad in patients makes such approach impractical in clinic.

To overcome the inherent limitations of a standard treatment regimen for multifocal HCC and oncolytic therapy, a cell sheet was studied as a promising candidate to enhance the efficacy of oncolytic adenoviruses in multifocal HCC.

Traditionally, a cell sheet has been mainly used in tissue engineering to replace or restore the function of damaged tissue. The main strengths of the cell sheet are biocompatibility, the ability of inducing durable engraftment, and a lower risk of adverse inflammatory responses than a synthetic antibody. However, no research on using such cell sheet in gene delivery has been reported.

### [Disclosure]

### [Technical Problem]

To address problems of systemic administration of a gene delivery system, the inventors developed a cell sheet as a local gene delivery platform, and thus the present invention was completed.

### [Technical Solution]

The present invention provides a cell sheet, which includes two or more cell layers,
wherein a gene delivery system is introduced into one or more of the layers.

In addition, the present invention provides a method of preparing a cell sheet, which includes
forming a cell sheet including two or more cell layers on a temperature-responsive culture dish including a temperature-responsive polymer,
introducing a gene delivery system to one or more of the cell layers, and
separating the cell sheet from the temperature-responsive culture dish.

In addition, the present invention provides a gene therapeutic agent including the cell sheet.

In addition, the present invention provides a method of preventing cancer recurrence or treating cancer, which includes transplanting the cell sheet on a cancer-removed site or a site in need of cancer treatment.

### [Advantageous Effects]

A cell sheet according to the present invention can be utilized as a local gene delivery system, unlike a cell sheet conventionally used for tissue regeneration. Particularly, when a virus is used as a gene delivery system, the virus can be proliferated in the cell sheet, and act locally on only a treatment lesion(site). Therefore, compared with systemic administration of viruses or intratumoral administration, even when a dose of viruses is considerably lowered, the cell sheet according to the present invention can be effectively used for prevention or treatment of cancer, recurrence of cancer or prevention of cancer metastasis, and particularly, treatment of multifocal tumors.

### [Description of Drawings]

FIG. 1 shows (a) the appearance of oAd-DCN/CFCSs separated from a temperature-responsive culture dish, (b) the histological analysis result of oAd-DCN/CFCSs by hematoxylin and eosin staining, (c) detection of the Ad E1A protein in PBS-treated control CFCSs, and (d) detection of the Ad E1A protein in oAd-DCN/CFCSs [Scale bars: 1cm (a) and 2µm (b-d)].
FIG. 2 show the viral production and therapeutic gene expression profile of oAd-DCN/CFCSs: [(a) Viral production of oAd-DCN infected into CFCSs. CFCSs were infected with naked oAd-DCN at 0.5 MOI. At 4, 12, 24, 48, 72 and 96 hours after infection, CFCSs and supernatants were harvested and then viral genome copies were measured by real-time quantitative PCR. (b) DCN expression in oAd-DCN/CFCSs. The representative western blot of DCN using cell lysates and supernatants harvested at 48 hours after infection with oAd-DCN at 1 MOI. Data was expressed as mean±SD. * P <0.05, ** P <0.01].

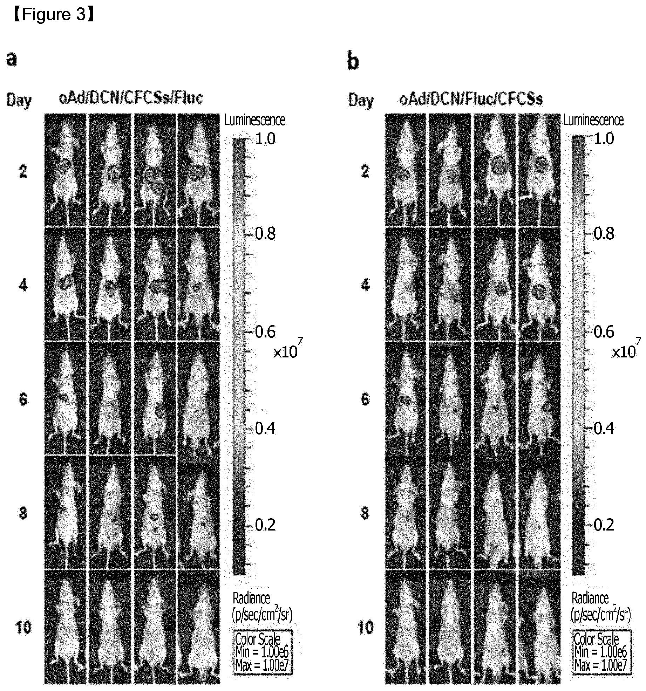

FIG. 3 shows the degradation profile and assessment of viral persistence of oAd-DCN/CFCSs *in vivo* after intrahepatic transplantation [(a) The degradation profile of a cell sheet. CFCSs were prepared with fibroblasts and firefly luciferase-expressing cancer cells (CFCSs/Fluc). Subsequently, CFCS/Fluc was infected with oAd-DCN, and then transplanted onto the left liver lobe of a nude mouse harboring an orthotopic hepatocellular carcinoma (HCC) tumor. (b) The assessment of viral persistence after transplantation of oAd-DCN/CFCSs. CFCSs were infected with firefly luciferase-expressing oAd-DCN (oAd-DCN/Fluc/CFCSs) and then transplanted onto the surface of the left lobe of a tumor-bearing mouse. Bioluminescence imaging was daily monitored after transplantation].
FIG. 4 shows the histological result of multifocal HCC [The cross-section of multifocal liver cancer tissue was obtained at 21 days after tumor cell injection from PBS-treated groups, and stained with hematoxylin and eosin. Original magnification: ×40, white dot line: tumor].
FIG. 5 shows the potent antitumor efficacy of oAd-DCN/CFCSs;
FIG. 5A shows the antitumor efficacy of oAd-DCN/CFCSs in a Hep3B orthotopic tumor model [An orthotopic liver tumor was established by injecting 1 x 10⁶ firefly luciferase-expressing Hep3B cells into the left liver lobe of a mouse. Immediately after the cell injection, the cell-injected site was treated with PBS, 3 × 10⁸ VP oAd-DCN (sprayed) by spraying, or transplanted with PBS-treated CFCSs or oAd-DCN/CFCSs (n= 6). In addition, 6 mice were systemically injected with 3 × 10⁸ VP into tail veins after Hep3B cell injection. Tumor growth was monitored on day 2, 5, 7, 14 and 21 after treatment].
FIG. 5B shows bioluminescent signals from HCC in treated groups after background subtraction [Data was expressed as mean±SD. * P <0.05].
FIG. 6 shows the histological analysis results of tumor tissues of mice each treated with PBS, oAd-DCN intravascular injection, oAd-DCN intraperitoneal injection, CFCSs only or oAd-DCN/CFCSs [The cross-sections of the treated multifocal HCC were obtained on day 14 after treatment with PBS, oAd-DCN intravascular injection, oAd-DCN intraperitoneal injection, CFCSs only or oAd-DCN/CFCSs. Original magnification: ×50, black dot line: tumor].
FIG. 7 is a schematic diagram of a process of forming oAd-DCN/CFCSs.
FIG. 8 shows the biological activity of an adenovirus replicated from an oncolytic adenovirus-loaded cell sheet.
FIG. 9 shows the recurrence of tumors after an oncolytic adenovirus-loaded cell sheet is attached following tumor resection.;
FIG. 10 shows the formation of a vaccinia virus-loaded cell sheet.
FIG. 11 shows the vaccinia viral replication ability of a vaccinia virus-loaded cell sheet.
FIG. 12 shows the vaccinia viral cell death of a vaccinia virus-loaded cell sheet.
FIG. 13 shows the cell viability of irradiated cancer cells.
FIG. 14 shows the adenoviral replication ability of a cell sheet in which irradiated cancer cells are loaded.

### [Best Mode]

The present invention provides a cell sheet, which includes two or more cell layers, wherein a gene delivery system is introduced into one or more of the layers.

In the present invention, the cell sheet is used for local gene delivery. That is, the cell sheet may serve as a type of carrier that may locally deliver a gene delivery system to a site in need of gene therapy.

The cell sheet may have mechanical properties suitable for handling by a user during preparation and *in vivo* transplantation.

To this end, as one of the cell layers, the cell sheet includes a cell layer acting as a support.

In one embodiment, as a support, the cell sheet includes a cell layer containing somatic cells.

The cell layer containing somatic cells serves as a support of a cell layer to which a gene delivery system is introduced, and are similar to in-vivo environment and may provide an environment which may allow attachment, proliferation, differentiation and culture of various cells.

Any type of somatic cell suitable for this role may be used, and therefore the type of the cell is not particularly limited. Specifically, the somatic cells may be one or more selected from the group consisting of fibroblasts, chondrocytes, epithelial cells, myoepithelial cells, dermal cells, epithelial keratinocytes, Schwann cells, glial cells, osteoblasts, cardiomyocytes, megakaryocytes, adipocytes, stem cells (e.g., mesenchymal stem cells) and cancer cells, but the present invention is not limited thereto.

In the present invention, any gene delivery system known to be used for gene therapy can be used.

For example, the gene delivery system of the present invention may be in the form of (i) a naked recombinant DNA molecule, (ii) a plasmid, (iii) a viral vector, and (iv) a liposome or niosome containing the naked recombinant DNA molecule or plasmid.

Any of the gene delivery systems used for typical gene therapy may be applied to the cell sheet according to the present invention, and is preferably plasmids, adenoviruses (Lockett LJ, et al., Clin. Cancer Res. 3:2075-2080(1997)), adeno-associated viruses (AAV, Lashford LS., et al., Gene Therapy Technologies, Applications and Regulations Ed. A. Meager, 1999), retroviruses (Gunzburg WH, et al., Retroviral vectors. Gene Therapy Technologies, Applications and Regulations Ed. A. Meager, 1999), lentiviruses (Wang G. et al., J. Clin. Invest. 104(11):R55-62(1999)), Herpes simplex virus (Chamber R., et al., Proc. Natl. Acad. Sci USA 92:1411-1415(1995)), Vaccinia virus (Puhlmann M. et al., Human Gene Therapy 10:649-657(1999)), a liposome (Methods in Molecular Biology, Vol 199, S.C. Basu and M. Basu (Eds.), Human Press 2002) or a niosome.

### i. Adenovirus

Adenoviruses are widely used as a gene delivery vector due to a medium-sized genome, easy handling, a high titer, a broad range of target cells and excellent infectivity. Both ends of the genome include 100 to 200-bp inverted terminal repeats (ITRs), respectively, which are cis elements required for DNA replication and packaging. The E1 region (E1A and E1B) of the genome encodes proteins regulating transcription and transcription of a host cell gene. The E2 region (E2A and E2B) encodes a protein involved in viral DNA replication.

Among currently-developed adenovirus vectors, E1 region-deficient incompetent adenoviruses are widely used. Meanwhile, the E3 region is removed from a typical adenovirus vector, and provides a site into which a foreign gene is inserted (Thimmappaya, B. et al., Cell, 31:543-551(1982); and Riordan, J. R. et al., Science, 245:1066-1073(1989)). Accordingly, a target nucleotide sequence to be delivered into a cell may be inserted into the deleted E1 region (E1A region and/or E1B region, preferably, E1B region) or E3 region, and preferably inserted into the deleted E1 region. The term "deletion" used herein in regard to a viral genome sequence means that the corresponding sequence is not only completely deleted, but also partially deleted.

An adenovirus has 42 different serotypes and A-F subgroups. Among them, adenovirus type 2 and type 5 belonging to the subgroup C is the most preferable starting material for obtaining the adenovirus vector of the present invention. Biochemical and genetic information for the adenovirus type 2 and type 5 are well known.

A foreign gene delivered by the adenovirus is replicated in the same manner as an episome, and thus has a very low genetic toxicity against a host cell. Accordingly, it is expected that the gene therapy using the adenovirus gene delivery system of the present invention will be very safe.

### ii. Retrovirus

A retrovirus has been widely used as a gene transfer vector, because its gene is inserted into the genome of a host, and it may deliver a large amount of foreign genetic materials and infect a wide spectrum of cells.

To construct a retrovirus vector, a desired nucleotide sequence to be delivered into a cell is inserted into a retroviral genome instead of a retroviral sequence to produce a replication-incompetent virus. To produce a virion, a packaging cell line (Mann et al., Cell, 33:153-159(1983)), which includes gag, pol and env genes, but not a long terminal repeat (LTR) and Ψ sequence, is constructed. When a recombinant plasmid including a target nucleotide sequence to be delivered, a LTR and a Ψ sequence is introduced into the cell line, the Ψ sequence allows the production of an RNA transcript of the recombinant plasmid, this transcript is packaged into a virus, and the virus is released into a medium (Nicolas and Rubinstein "Retroviral vectors," In: Vectors: A survey of molecular cloning vectors and their uses, Rodriguez and Denhardt (eds.), Stoneham: Butterworth, 494-513(1988)). The medium containing the recombinant retroviruses is collected and concentrated to be used as a gene delivery system.

Gene transfer using a second-generation retrovirus vector was suggested. According to Kasahara et al. Science, 266:1373-1376(1994), a mutant of Moloney murine leukemia virus (MMLV) was prepared, and here, an erythropoietin (EPO) sequence was inserted into an envelope site to produce a chimeric protein having new binding properties. The gene delivery system of the present invention may also be prepared according to the construction strategy of the second-generation retrovirus vector.

### iii. AAV vector

An adeno-associated virus (AAV) is suitable as a gene delivery system of the present invention because they infect non-dividing cells and having the ability to transfect various types of cells. Detailed descriptions of the manufacturing and use of the AAV vector are disclosed in detail in U.S. Patent Nos. 5,139,941 and 4,797,368.

Studies on AAVs as a gene delivery system are disclosed in LaFace et al, Viology, 162:483486(1988), Zhou et al., Exp. Hematol. (NY), 21:928-933(1993), Walsh et al, J. Clin. Invest., 94:1440-1448(1994) and Flotte et al., Gene Therapy, 2:29-37 (1995).

Typically, AAVs are manufactured by co-transforming a plasmid (McLaughlin et al., J. Virol., 62:1963-1973(1988); and Samulski et al., J. Virol., 63:3822-3828(1989)) including a desired gene sequence (a desired nucleotide sequence to be delivered into a cell) flanked by two AAV terminal repeats, and an expression plasmid (McCarty et al., J. Virol., 65:2936-2945(1991)) including a wild-type AAV coding sequence without a terminal repeat.

### iv. Other viral vectors

Other viral vectors may also be used as the gene delivery system of the present invention. Vectors derived from vaccinia virus (Puhlmann M. et al., Human Gene Therapy 10:649-657(1999); Ridgeway, "Mammalian expression vectors," In: Vectors: A survey of molecular cloning vectors and their uses. Rodriguez and Denhardt, eds. Stoneham: Butterworth, 467-492(1988); Baichwal and Sugden, "Vectors for gene transfer derived from animal DNA viruses: Transient and stable expression of transferred genes," In: Kucherlapati R, ed. Gene transfer. New York: Plenum Press, 117-148(1986) and Coupar et al., Gene, 68:1-10(1988)), lentiviruses (Wang G. et al., J. Clin. Invest. 104(11):R55-62(1999)) or Herpes simplex viruses (Chamber R., et al., Proc. Natl. Acad. Sci USA 92:1411-1415(1995)) may also be used as a delivery system which may deliver a desired nucleotide sequence into a cell.

Other than these, viral vectors include reoviruses, poxviruses, Semliki forest viruses and Measles viruses, etc.

### v. Liposome

Liposomes are automatically formed by phospholipids dispersed in an aqueous phase. Examples of successful delivery of foreign DNA molecules into cells using liposomes are disclosed in Nicolau and Sene, Biochim. Biophys. Acta, 721:185-190 (1982) and Nicolau et al., Methods Enzymol., 149:157-176 (1987). Meanwhile, Lipofectamine (Gibco BRL) is the most widely used reagent for transformation of animal cells using liposomes. Liposomes containing a target nucleotide sequence to be delivered interact with cells by a mechanism such as endocytosis, adsorption onto a cell surface or fusion with a plasma cell membrane to deliver a target nucleotide sequence into cells.

A method of introducing a gene delivery system into one or more cell layers is performed by bringing cells constituting a cell layer into a contact with the gene delivery system.

In the present invention, when a gene delivery system is manufactured based on a viral vector, the contacting step is performed by a virus infection method known in the art. The infection of host cells using a virus vector is described in the references cited above.

In the present invention, when the gene delivery system is a naked recombinant DNA molecule or plasmid, a gene may be introduced into cells by microinjection (Capecchi, M.R., Cell, 22:479(1980); and Harland and Weintraub, J. Cell Biol. 101:1094-1099(1985)), calcium phosphate precipitation (Graham, F.L. et al., Virology, 52:456(1973); and Chen and Okayama, Mol. Cell. Biol. 7:2745-2752(1987)), electroporation (Neumann, E. et al., EMBO J., 1:841(1982); and Tur-Kaspa et al., Mol. Cell Biol., 6:716-718(1986)), liposome-mediated transfection (Wong, T.K. et al., Gene, 10:87(1980); Nicolau and Sene, Biochim. Biophys. Acta, 721:185-190(1982); and Nicolau et al., Methods Enzymol., 149:157-176(1987)), DEAE-dextran treatment (Gopal, Mol. Cell Biol., 5:1188-1190(1985)), and gene bombardment (Yang et al., Proc. Natl. Acad. Sci., 87:9568-9572(1990)).

In one embodiment of the present invention, the cell sheet according to the present invention is used as a local delivery platform which enables viral replication. The cell sheet allows oncolytic viruses to cover a target tumor site, expression of a specific gene (e.g., decorin) favorable for tumor removal, and effective replication of oncolytic viruses and therapeutic genes. In addition, long-term persistence of oncolytic viruses in tumor tissue is possible by allowing the active replication of virions in both of the cell sheet and tumor tissue until the cell sheet is completely degraded in the body.

According to this, the cell sheet elicits a more potent antitumor effect than the conventional intratumorally-administered oncolytic virus, and effectively prevents multifocal carcinogenesis. In addition, since the cell sheet-mediated delivery of oncolytic viruses is localized in a tumor site, non-specific release of virions into normal tissue is prevented. As the administration of oncolytic viruses loaded on the cell sheet simultaneously reinforces intratumoral localization and non-specific release of the viruses is prevented, the therapeutic efficacy of viruses may be prolonged, amplified and strengthened as well as a safety profile may be enhanced.

In one embodiment of the present invention, the gene delivery system may be a recombinant adenovirus.

As various advantages of a recombinant adenovirus as a gene delivery vector are highlighted, the frequency of its use in cancer gene therapy is steadily increasing. Particularly, when cancer is to be treated with a gene therapeutic agent, there is no need of long-term and continuous expression of a therapeutic gene. In addition, since the immune response of a host induced by a virus used as a vector is not problematic or rather can act as an advantage, a recombinant adenovirus attracts attention as a gene carrier for cancer treatment.

The recombinant adenovirus may be a replication-incompetent adenovirus or oncolytic adenovirus.

The replication-incompetent adenovirus is recombined by inserting a therapeutic gene instead of an E1 gene (total or a part) required for the replication of the adenovirus, and designed so as not to be replicated in adenovirus-introduced cells.

An oncolytic adenovirus is an adenovirus from which anElB 55kDa gene is partially deleted, and can be proliferated only in cells in which p53 is functionally inactivated. In cancer cells in which the function of p53 is suppressed, viral proliferation actively occurs, but in normal cells, viral proliferation is inhibited. Therefore, an oncolytic adenovirus does not affect normal cells and selectively kills cancer cells, which is particularly advantageous for cancer treatment.

In one embodiment of the present invention, a recombinant adenovirus may have an inactivated E1B 19kDa gene, E1B 55kDa gene or E1B 19kDa/ElB 55kDa gene, and preferably has inactivated E1B 19kDa and E1B 55kDa genes.

In the specification, the term "inactivation" used in connection with a gene means that, due to abnormal transcription and/or translation of the gene, normal functions of a protein encoded by the gene are not exhibited. For example, the inactivated E1B 19kDa gene is a gene that cannot produce an activated E1B 19 kDa protein because of a mutation (substitution, addition, partial deletion or complete deletion) in the gene. When the E1B 19kDa gene is absent, apoptosis may increase, and when the E1B 55kDa gene is absent, tumor cell specificity may be exhibited (refer to Korean Patent Application No. 2002-0023760).

According to one embodiment of the present invention, the recombinant adenovirus of the present invention may include an active E1A gene. The recombinant adenovirus having the E1A gene has a property of being able to replicate. According to a more preferable embodiment of the present invention, the recombinant adenovirus of the present invention includes the inactivated E1B 19kDa/ElB 55kDa gene and the active E1A gene. According to an embodiment of the present invention, in the recombinant adenovirus of the present invention, the E1B 19kDa/E1B 55kDa genes are deleted and the active E1A gene is included, and a decorin-encoding nucleotide sequence is inserted into the deleted E1 region.

In one embodiment of the present invention,
a gene delivery system-introduced cell layer may include cancer cells or stem cells.

When oncolytic adenoviruses are used as a gene delivery system, a cell layer to which a gene delivery system is introduced may include cancer cells. As described above, oncolytic adenoviruses are proliferated only in cancer cells, and the replication of oncolytic adenoviruses is inhibited in normal cells, rather than the cell layer including cancer cells. For this reason, even when the cell sheet is cultured for a long time, there is no need to worry about the degradation of mechanical properties due to death of the cell layer acting as a support. In addition, following *in vivo* transplantation of the cell sheet, since cancer cells themselves are killed by oncolytic adenoviruses, it is not necessary to worry about cancer cells remaining in the body.

Even though another gene delivery system, rather than oncolytic adenoviruses, is used, since cancer cells may lose their replication ability by radiation therapy, and therefore, there is no problem in using the cell sheet as a gene delivery system-introduced cell layer. In one embodiment of the present invention, the cell sheet includes a cell layer including cancer cells, to which a gene delivery system is introduced, and the cancer cells may have been irradiated.

The cancer cells may be, specifically, cancer cells derived from one or more types of cancer selected from the group consisting of glioblastoma, laryngeal cancer, pancreatic cancer, lung cancer, non-small cell lung cancer, colon cancer, bone cancer, skin cancer, head and neck cancer, ovarian cancer, uterine cancer, rectal cancer, gastric cancer, anal cancer, colorectal cancer, breast cancer, fallopian cancer, endometrial cancer, cervical cancer, vaginal cancer, vulva cancer, Hodgkin's disease, esophageal cancer, small intestine cancer, endocrine gland tumors, thyroid cancer, parathyroid carcinoma, adrenal cancer, soft tissue sarcoma, urethral cancer, penile cancer, prostate cancer, chronic or acute leukemia, lymphocyte lymphoma, bladder cancer, kidney or urinary tract cancer, renal cell carcinoma, renal pelvic carcinoma, central nervous system (CNS) tumors, primary CNS lymphoma, spinal tumors, liver cancer, bronchial cancer, nasopharyngeal cancer, brainstem glioma and pituitary adenoma, but the present invention is not limited thereto.

In another embodiment, the gene delivery system-introduced cell layer may include stem cells. Since the stem cells have a tumor targeting ability, it may be particularly advantageous that the cell sheet of the present invention is used for cancer patients. However, generally, since stem cells have been known to have a low infection rate of adenoviruses, recombinant adenoviruses with an enhanced stem cell introduction ability are preferably used. When the gene delivery system-introduced cell layer includes stem cells, recombinant adenoviruses with an enhanced ability of introduction into stem cells including the serotype 35 fiber knob are preferably used, but the present invention is not limited thereto. The recombinant adenoviruses including the serotype 35 fiber knob have significantly excellent efficiency of introduction into mesenchymal stem cells, and highly-effective introduction even at a low viral concentration.

In one embodiment of the present invention, the recombinant adenovirus of the present invention may have an inactivated E1B 19kDa gene, E1B 55kDa gene or E1B 19kDa/ElB 55kDa gene, and preferably, inactivated E1B 19kDa and E1B 55kDa genes, but the present invention is not limited thereto.

The term "inactivation" used herein in regard to a gene means that, due to abnormal transcription and/or translation of the gene, normal functions of a protein encoded by the gene may not be exhibited. For example, the inactivated E1B 19kDa gene is a gene that cannot produce activated E1B 19 kDa protein because of a mutation (substitution, addition, partial or complete deletion) on a gene. When the E1B 19kDa gene is absent, apoptosis may increase, and when the E1B 55kDa gene is absent, tumor cell specificity is exhibited (refer to Korean Patent Application No. 2002-0023760).

According to a preferable embodiment of the present invention, the recombinant adenovirus of the present invention includes an active E1A gene. The recombinant adenovirus having the E1A gene has a property of being able to replicate. According to a more preferable embodiment of the present invention, the recombinant adenovirus of the present invention includes the inactivated E1B 19kDa/ElB 55kDa gene and the active E1A gene. According to the most preferable embodiment of the present invention, in the recombinant adenovirus of the present invention, the E1B 19kDa/ElB 55kDa genes are deleted, and the active E1A gene is included, and a decorin-encoding nucleotide sequence is inserted into the deleted E1 region.

The recombinant adenovirus used in the present invention includes a promoter that is operable in animal cells, and preferably, mammal cells. Promoters suitable for the present invention include promoters derived from mammalian viruses and promoters derived from genomes of mammalian cells, for example, a cytomegalovirus (CMV) promoter, an U6 promoter and a H1 promoter, a murine leukemia virus (MLV) long terminal repeat (LTR) promoter, an adenovirus early promoter, an adenovirus post promoter, a vaccinia virus 7.5K promoter, a SV40 promoter, a HSV tk promoter, an RSV promoter, an EF1 α promoter, a metallothionein promoter, a β-actin promoter, a human IL-2 gene promoter, a human IFN gene promoter, a human IL-4 gene promoter, a human lymphotoxin gene promoter, a human GM-CSF gene promoter, an inducible promoter, a cancer cell-specific promoter (e.g., a TERT promoter, a modified TERT promoter, a PSA promoter, a PSMA promoter, a CEA promoter, a Survivin promoter, an E2F promoter, a modified E2F promoter, an AFP promoter, a modified AFP promoter, an E2F-AFP hybrid promoter, or an E2F-TERT hybrid promoter), a tissue-specific promoter (e.g., an albumin promoter), a human phosphoglycerate kinase (PGK) promoter, and a mouse phosphoglycerate kinase (PGK) promoter, but the present invention is not limited thereto. Most preferably, the promoter suitable for the present invention is a CMV promoter. In an expression construct for expressing a transgene, a polyadenylation sequence is preferably linked downstream of a transgene. The polyadenylation sequence is a bovine growth hormone terminator (Gimmi, E. R., et al., NucleicAcids Res. 17:6983-6998(1989)), an SV40-derived polyadenylation sequence (Schek, N, et al., Mol. Cell Biol. 12:5386-5393(1992)), HIV-1 polyA (Klasens, B. I. F., et al., Nucleic Acids Res. 26:1870-1876(1998)), β globin polyA (Gil, A., et al, Cell 49:399-406(1987)), HSV TK polyA (Cole, C. N. and T. P. Stacy, Mol. Cell. Biol. 5:2104-2113(1985)), or polyomavirus polyA (Batt, D. B and G. G. Carmichael, Mol. Cell. Biol. 15:4783-4790(1995)), but the present invention is not limited thereto.

The recombinant adenovirus of the present invention may further include an antibiotic resistant gene and a reporter gene (e.g., a green fluorescence protein (GFP), luciferase and β-glucuronidase) as a selective marker. The antibiotic resistant gene includes genes which are resistant to antibiotics conventionally used in the art, for example, genes resistant to ampicillin, gentamicin, carbenicillin, chloramphenicol, streptomycin, kanamycin, geneticin, neomycin and tetracycline. Genes resistant to neomycin is preferable.

A viral vector (e.g., recombinant adenovirus) loaded in the cell sheet according to the present invention is contained at 0.1 to 500 multiplicity of infection (MOI). More preferably, the viral vector is loaded at 0.1 to 200 MOI, 0.1 to 100 MOI, 0.1 to 50 MOI, 0.1 to 10 MOI, 0.1 to 5 MOI, 0.5 to 200 MOI, 0.5 to 100 MOI, 0.5 to 50 MOI, 0.5 to 10 MOI or 0.5 to 5 MOI. Since the viral vector can be proliferated in cancer cells even at a considerably lower amount than 1 × 10¹⁰ VP to 5 × 10¹⁰ VP, which is an amount of oncolytic viruses used in a conventional tumor therapy, a virus loading amount may be significantly lowered and a burden that doctors can feel may be significantly reduced, compared with the conventional tumor therapy using oncolytic viruses.

In addition, the present invention provides a method of preparing a cell sheet, which includes
forming a cell sheet including two or more cell layers on a temperature-responsive culture dish including a temperature-responsive polymer,
introducing a gene delivery system to one or more of the cell layers, and
separating the cell sheet from the temperature-responsive culture dish.

All the contents described above in regard to the cell sheet may be applied as is or applied correspondingly to a method of preparing a cell sheet.

The method of preparing a cell sheet according to the present invention may include the following steps, but the present invention is not limited thereto:
forming a first cell layer including somatic cells by culturing the somatic cells in a temperature-responsive culture dish containing a temperature-responsive polymer;
preparing a cell sheet by forming a second cell layer including cancer cells by culturing the cancer cells on the first cell layer;
inoculating the second cell layer with oncolytic viruses; and
separating the cell sheet from the temperature-responsive culture dish.

Specifically,
first, a first cell layer including somatic cells is formed by placing a silicone ring on a temperature-responsive culture dish containing a temperature-responsive polymer, and seeding the somatic cells serving as a support inside the silicone ring and culturing the cells in a constant temperature unit.

The temperature-responsive culture dish may be used to form a cell sheet by attaching cells to the surface thereof at a lower critical solution temperature (LCST) or more and be used to collect cells in a sheet form by swelling a polymer at LCST or less.

The temperature-responsive polymer may be one or more selected from the group consisting of poly(N-isopropylacrylamide), poly(N-vinylcaprolactame), polycaprolactone (PCL) and polylactate-co-glycolate (PLGA), but any polymer with temperature responsiveness may be used without limitation.

Subsequently, a cell sheet is prepared by forming a second cell layer including cancer cells by seeding the cancer cells on the first cell layer and culturing the cells in a constant temperature unit.

Particularly, the method of preparing a cell sheet may further include irradiating the second cell layer to remove the possibility of carcinogenesis caused by the cancer cells constituting the cell sheet.

Subsequently, viruses are loaded on the cell sheet by inoculating the second cell layer with oncolytic viruses.

Here, the oncolytic viruses are inoculated at 0.1 to 500 MOI (more preferably 0.1 to 200 MOI, 0.1 to 100 MOI, 0.1 to 50 MOI, 0.1 to 10 MOI, 0.1 to 5 MOI, 0.5 to 200 MOI, 0.5 to 100 MOI, 0.5 to 50 MOI, 0.5 to 10 MOI or 0.5 to 5 MOI) at 12 hours to 1 day after the formation of the cell sheet. The sheet may be formed by inoculation of cells at regular intervals, and a loading amount of viruses per number of cells is able to be calculated. By the inoculation of the oncolytic viruses, cancer cells are naturally killed after a certain period (12 hours to 7 days after inoculation). In addition, since the oncolytic viruses can be amplified by cancer cells, the cancer cells can be treated with a small amount of the oncolytic viruses.

Finally, the cell sheet is separated from the temperature-responsive culture dish.

Since a polymer of the temperature-responsive culture dish is swollen at LCST or less, the cell sheet may be detached from the culture dish. Here, at 6 hours to 1 day after viral inoculation, the separation of the cell sheet from the culture dish is preferable because the degradation of the cell sheet caused by the replication of the loaded viruses may not occur, and the cell sheet may be detached in the form of a solid cell sheet.

In addition, the present invention provides a gene therapeutic agent which includes the cell sheet of the present invention as an active ingredient.

The term "gene therapeutic agent" used herein refers to cells or a medicine which allows administration of a genetic material or a genetic material-harboring carrier into a subject for the purpose of disease treatment. In addition, the gene therapeutic agent refers to a medicine used to treat or prevent a genetic defect by injecting a normal gene or gene having a therapeutic effect into a damaged gene of a subject.

A pharmaceutically acceptable carrier which can be applied as a gene therapeutic agent is sterile and biocompatible, and may be saline, sterile water, Ringer's solution, buffered saline, an albumin injection solution, a dextrose solution, a maltodextrin solution, glycerol, ethanol or a mixture of one or more thereof, and as needed, other conventional additives such as an antioxidant, a buffer solution and a bacteriostatic agent may be added. In addition, by additionally adding a diluent, a dispersing agent, a surfactant, a binder and a lubricant, the pharmaceutically acceptable carrier may be prepared as an injectable formulation such as a solution, a suspension or an emulsion, a pill, a capsule, a granule or a tablet, and may be used by linking the carrier with a target organ-specific antibody or another ligand to specifically act on a target organ.

Preferably, the gene therapeutic agent of the present invention may be used to prevent or treat cancer, or prevent cancer recurrence or metastasis.

The cancer may be one or more selected from the group consisting of multifocal hepatocellular carcinoma (HCC), glioma, glioblastoma, laryngeal cancer, pancreatic cancer, lung cancer, non-small cell lung cancer, colon cancer, bone cancer, pancreatic cancer, skin cancer, head and neck cancer, ovarian cancer, uterine cancer, rectal cancer, gastric cancer, anal cancer, colorectal cancer, breast cancer, fallopian cancer, endometrial cancer, cervical cancer, vaginal cancer, vulva cancer, Hodgkin's disease, esophageal cancer, small intestine cancer, endocrine gland tumors, thyroid cancer, parathyroid carcinoma, adrenal cancer, , soft tissue sarcoma, urethral cancer, penile cancer, prostate cancer, chronic or acute leukemia, lymphocyte lymphoma, bladder cancer, kidney or urinary tract cancer, renal cell carcinoma, renal pelvic carcinoma, central nervous system (CNS) tumors, primary CNS lymphoma, spinal tumors, liver cancer, bronchial cancer, nasopharyngeal cancer, brainstem glioma and pituitary adenoma.

Cancer may be treated or the cancer metastasis or recurrence may be prevented by administering the cell sheet of the present invention to a cancer (tumor)-removed site or a cancer-occurring site. A preferable dose of the cell therapeutic agent of the present invention may vary according to the condition and body weight of a subject, the severity of a disease, a dosage form, an administration route and an administration period, and may be properly selected by those of ordinary skill in the art. The administration may be performed once or several times a day, and the dose does not limit the scope of the present invention in any way.

The term "prevention" used herein refers to all actions of inhibiting cancer (tumor) or delaying the onset thereof by administration of the cell sheet according to the present invention.

The term "treatment" used herein refers to all actions involved in alleviating or beneficially changing symptoms of cancer (tumor) by administration of the cell sheet according to the present invention.

The term "metastasis" used herein refers to a condition in which malignant tumors spread to different tissues apart from an organ in which the malignant tumors have occurred.

The term "recurrence" used herein refers to the case in which a tumor has disappeared by surgical removal or radiation therapy and then the same tumor develops, the case in which remaining tumor cells are proliferated, or the case in which tumor cells are thoroughly removed and then a tumor develops.

In addition, the present invention provides a method of preventing cancer recurrence, which includes transplanting a cell sheet according to the present invention onto a cancer-removed site of a subject.

In addition, the present invention provides a method of treating cancer, which includes transplanting a cell sheet according to the present invention onto a site in need of cancer treatment of a subject.

The term "subject" used herein refers to a target in need of treatment, and more specifically, a mammal such as a human or a non-human primate, a rodent (a rat, a mouse or a guinea pig), a dog, a cat, a horse, a cow, sheep, a pig, a goat, a camel or an antelope.

In the cell sheet according to the present invention, at a certain period (6 days or 10 days) after transplantation, a cancer cell layer naturally disappears due to destroy by viral replication.

A method of transplanting the cell sheet of the present invention onto a target site may include, for example, the following steps, but the present invention is not limited thereto:

A culture medium is removed from the cell sheet infected with viruses formed in a temperature-responsive culture dish, and the cell sheet is washed with PBS. While a membrane is placed on the cell sheet, when the cell sheet is lowered in temperature and then detached, the cell sheet attached to the membrane may be obtained. When the cell sheet-attached membrane is placed on a target site (lesion), and then the membrane is carefully detached, the cell sheet is attached to the target site, and by removing the membrane, the cell sheet can be transplanted onto the target site.

Hereinafter, the present invention will be described in detail through the Examples. However, the following Examples are only for exemplifying the present invention, and the scope of the present invention is not limited to the following Examples.

### [Examples]

### Experimental Methods

### Cell lines and cell culture

All cell lines were cultured in Dulbecco's modified Eagle's medium (DMEM, Gibco BRL, Grand Island, NY USA) supplemented with 10% fetal bovine serum (FBS, Gibco BRL) and penicillin-streptomycin (100 IU/mL, Gibco BRL).

HEK293 (human embryonic kidney cell line expressing the Ad E1 region), A549 (lung cancer cell line), Hep3B (hepatocellular carcinoma cell line), U343 (glioblastoma cell line) and NIH3T3 (fibroblast cell line) cell lines were purchased from the American Type Culture Collection (ATCC, Manassas, VA).

All cell lines were maintained at 37 °C in a humidified atmosphere containing 5% CO₂.

### Manufacture of decorin-expressing oncolytic adenovirus (oAd-DCN)

A DCN-expressing cassette was obtained by cleaving pCA14/DCN using the Bgl II restriction enzyme, and ligated with pdE1sp1B(p)-HRE-mTERT-Rd19 cut with the BamHI restriction enzyme, thereby manufacturing a pdE1sp1B(p)-HRE-mTERT-Rd19/DCN E1 shuttle vector.

The vector was treated with the *Xmn*I restriction enzyme to make it a single strand, and dl324-k35, which is a vector prepared by substituting an adenovirus knob with that of Ad35, was treated with the *Bst*BI restriction enzyme to make it a single strand. And then, the two vectors were simultaneously transformed into E. coli BJ5183 to induce gene homologous recombination, thereby manufacturing DCN-expressing oAd vectors (HmT-Rd19-k35/DCN, oAd-DCN).

### Adenovirus-loaded cancer cell/fibroblast cell sheets (oAd/CFCSs)

A cell sheet (CFCS) composed of a first cell layer of fibroblasts and a second cell layer of cancer cells was prepared using a temperature-responsive culture dish (TRCD; UpCell; NUNC, Tokyo, Japan).

To form a double-layered cell sheet, NIH3T3 cells (3 × 10⁵) were seeded in a hollow inner layer of a silicone ring (radius: 1.8 cm) on a 35mm TRCD and cultured at 37 °C. After 48 hours of the culture, U343 cells (3 × 10⁵) were added to a silicone ring-confined area, thereby forming a second layer of the cell sheet and incubated for 24 hours. The medium in each dish was exchanged with fresh DMEM containing 5% FBS, and then the cells were infected with decorin-expressing oncolytic adenoviruses (oAd-DCN) at 0.5 or 5 MOI. Ad-DCN-infected cancer cell/fibroblast cell sheets (oAd-DCN/CFCSs) were detached from the dish at 4 hours after infection by lowering the culture temperature to room temperature for 30 minutes.

### Histology

oAd-DCN CFCS was prepared as described above using 5 MOI of oAd-DCN, and control CFCS was prepared by treating the cell sheet with phosphate buffered saline (PBS).

Oncolytic adenovirus-loaded or PBS-treated CFCS was harvested at 12 hours after treatment by lowering a culture temperature to room temperature for 30 minutes and then fixing the cell sheet with 4% paraformaldehyde for 24 hours. The fixed samples were embedded in paraffin, cut into a 5 µm cross-section, and deparaffinized for hematoxylin and eosin (H&E) staining.

### Viral production assay

To evaluate the viral production of oncolytic adenoviruses in a cell sheet, CFCSs were placed in a 12-well plate and infected with oAd-DCN at 0.5 MOI. Four hours after infection, the wells were washed with PBS, and a medium was exchanged with fresh DMEM containing 5% FBS.

At 4, 12, 24, 48, 72 and 96 hours after infection, both of a supernatant and the cell sheet were collected, and the number of adenovirus particles was assessed by real-time quantitative PCR (Q-PCR, TaqMan PCR detection, Applied Biosystems, CA, USA).

### Western blotting

To evaluate the level of oncolytic adenovirus-mediated DCN expression in oAd-DCN/CFCSs, the sheet was infected with oAd-DCN at 1 MOI and incubated for 48 hours. Subsequently, the sheet was homogenized in an ice-cold RIPA buffer (Elipis Biotech, Taejeon, Korea) containing a proteinase inhibitor cocktail (Sigma, MO, USA), and the resulting homogenate was centrifuged for 10 minutes at 13,200 rpm.

A total protein concentration was measured by a BCA protein assay (Pierce, Rockford, IL, USA), and an equal amount of a protein (200 µg per sample) was loaded on a sodium dodecyl sulfate-polyacrylamide gel for electrophoresis. The protein was transferred to a polyvinylidene fluoride membrane, and incubated with goat anti-DCN Ab (Ab, R & D Systems, MN, USA) or rabbit anti-β-actin antibody (Cell Signaling Technology, Beverly, MA, USA).

The membrane was incubated with horseradish peroxidase-conjugated mouse anti-goat IgG Ab or goat anti-rabbit IgG (Cell Signaling) as secondary Ab, and an immunoreactive band was visualized by enhanced chemiluminescence (Amersham Pharmacia Biotech, Uppsala, Sweden).

The expression level of DCN was semi-quantitatively analyzed using ImageJ software (National Institutes of Health, Bethesda, MD, USA).

### MTT assay

To assess the viral replication-mediated degradation in vitro, CFCSs were placed in 48-well plate and infected with oAd-DCN at 5 MOI.

At 4, 12, 24, 48, 72 and 96 hours after infection, a medium was removed and CFCSs were treated with 500 µL of 3-(4,5-dimethylthiazol-2-yl)-2,5-diphenyltetrazolium bromide (MTT, Sigma, MO, USA). Subsequently, the plate was read with a microplate reader at 540 nm. The absorbance from a PBS-treated group was set as 100% viability.

### In vivo degradation profile and viral persistence of oAd-DCN/CFCSs

To assess the degradation and viral persistence of CFCSs *in vivo,* two different types of cell sheets were prepared.

The degradation profile of CFCSs was assessed by using CFCSs composed of fibroblasts and firefly luciferase (fluc)-expressing cancer cells (CFCSs/fluc), and infected with oAd-DCN at 5 MOI, thereby forming oAd-DCN/CFCSs/fluc.

To assess the viral persistence of a cell sheet, CFCSs were prepared using fibroblasts and cancer cells that do not express the fluc gene and infected with firefly luciferase-expressing oAd-DCN (oAd-DCN-fluc) at 5 MOI. At 4 hours after infection, all of oAd-DCN/CFCSs/fluc and oAd-DCN-fluc/CFCSs were transplanted onto the largest liver lobe of HCC-bearing mice. At 2, 4, 6, 8 and 10 days after transplantation, the mice were anesthetized in a chamber filled with 2% isoflurane in oxygen and intraperitoneally injected with D-luciferase (150 mg/kg, Caliper, Hopkinton, MA) to assess the degradation of oAd-DCN/CFCS/fluc and persistence of oncolytic adenoviruses in oAd-DCN-fluc/CFCSs using an IVIS imaging system (Xenogen, Alameda, CA, USA).

### In vivo antitumor efficacy

To assess the antitumor effect of oAd-DCN, CFCSs and oAd-DCN/CFCSs in an orthotopic HCC xenograft model 1x10⁶ fluc-expressing Hep3B cells (Hep3B/fluc) were injected into the largest lobe of the liver in 6 to 7 week-old athymic nude mice (OrientBio Inc., Seongnam, Korea).

Immediately after tumor cell injection, the injected site of the liver was treated with trypsin for 5 minutes, and then transplanted with a CFCS group (CFCS or oAd-DCN/CFCS) or sprayed with PBS or oAd-DCN (5 MOI). Finally, one of the treated groups was systemically administered oAd-DCN via a tail vein (at the same dose of viruses as other oncolytic adenovirus-containing groups).

Two days after cell injection, mice were anesthetized in a chamber filled with 2% isoflurane in oxygen, and intraperitoneally injected with D-luciferin (150 mg/kg; Caliper, MA, Hopkinton, MA) to confirm successful implantation of Hep3B/fluc cells using an IVIS imaging system (Xenogen).

*In vivo* bioluminescence signal intensities were obtained as photons per second ([p/s]) from a body region of interest (tumor) on day 2, 5, 7, 14 and 21 after treatment.

### Histological and immunohistochemical analyses

Hep3B-HCC tumor tissues were harvested at 21 days after HCC cell injection, fixed in 10% formalin, embedded in paraffin, and cut into 5 µm sections. Sections were stained with H&E and examined by optical microscopy. To detect adenovirus particles in tumor tissues, the tumor sections were immunostained with rabbit anti-Ad E1A polyclonal Ab (Santa Cruz Biotechnology).

In addition, the tumor sections were immunostained with proliferating cell nuclear antigen (PCNA)-specific Ab (Dako, Glostrup, Denmark) or by terminal deoxynucleotidyl transferase dUTP nick end labeling (TUNEL) to assess tumor cell proliferation or induction of apoptosis after treatment. Afterward, the tumor sections were treated with horseradish peroxidase-conjugated goat anti-rat IgG (BD Biosciences Pharmingen) or horseradish peroxidase-conjugated goat anti-mouse IgG (Southern Biotech, Birmingham, AL, USA) as a secondary antibody.

Diaminobenzidine/hydrogen peroxidase (DAKO) was used as a chromogen substrate. All slides were counterstained with Mayer's hematoxylin.

### Statistical analysis

Data was expressed as mean±SD. Statistical significance was measured by a two-tailed Student T-test or One-way Anova test (SPSS 13.0 software, SPSS, Chicago, IL). P values less than 0.05 were considered statistically significant.

### Confirmation of cell viability of irradiated cancer cells

After various intensities (1, 5, 10, 15, 30, and 50 Gy) of radiation were applied to a U343 cell line, and the resulting U343 cell line was seeded in a 96-well plate and then subjected to a MTT assay on day 9 of culture to confirm cell viability of the irradiated cancer cells.

### Confirmation of viral replication ability from cell sheet using irradiated cancer cells

A cell sheet (CFCS) composed of a first cell layer of fibroblasts and a second cell layer of cancer cells was prepared using a temperature-responsive culture dish (TRCD; UpCell; NUNC, Tokyo, Japan).

To form a double-layered cell sheet, NIH3T3 cells (3 × 10⁵) were seeded in a hollow inner layer of a silicone ring (radius: 1.8 cm) on a 35mm TRCD and cultured at 37 °C. After 48 hours of the culture, U343 cells which were not irradiated (control) or U343 cells (3 × 105) irradiated with 5 Gy were added to a silicone ring-confined area, thereby forming a second layer of the cell sheet and incubated for 24 hours. The medium in each dish was exchanged with fresh DMEM containing 5% FBS, and then the cells were infected with DCN-expressing oncolytic adenoviruses (oAd-DCNs) at 0.5 MOI. Afterward, to remove uninfected viruses at 4 hours after infection, the medium was exchanged with a fresh medium, and at 4, 24 and 48 hours, the culture medium and the cells were harvested to confirm viruses present therein by Q-PCR.

### Confirmation of biological activity and tumorigenesis after transplantation of cell sheet onto tumor removed (target) region

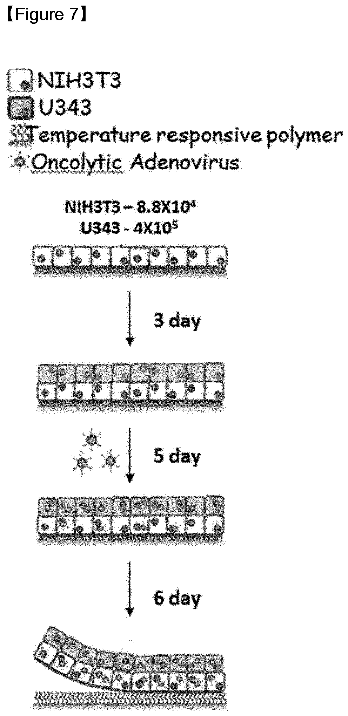

As shown in FIG. 7, NIH3T3 was seeded in a temperature-responsive culture dish to form a first layer, and after 3 days, a U343 cell line was seeded to form a second layer. After 48 hours, the cell sheet was infected with oncolytic adenoviruses, and after 24 hours, the temperature of the temperature-responsive culture dish was lowered to 20 °C to detach a cell sheet.

The culture medium was removed from the cell sheet infected with the viruses formed in the temperature-responsive culture dish, and then washed three times with 1× PBS. While a membrane was placed on the cell sheet, the temperature was lowered to 20 °C, thereby obtaining a cell sheet-attached membrane.

After a tumor in a H460 lung cancer cell tumor-bearing mouse model was removed by a surgical operation, the cell sheet-attached membrane was placed on a tumor-removed region, the membrane was gently detached, and then the cell sheet was attached to the tumor-removed region. Afterward, the surgical region was closed, and whether a tumor recurred was observed until day 30.

### Analysis of biological activity of virus produced in cell sheet

To assess the biological activity of oncolytic adenoviruses replicated in the cell sheet, CFCSs were added to a 12-well plate, and infected with 0.5 MOI of oAd-DCN. Four hours after infection, the well was washed with 1× PBS, and a medium was exchanged with fresh DMEM containing 5% FBS. At 4, 12, 24, 48, and 72 hours of after infection, both of a supernatant and the cell sheet were collected.

An A549 cell line was seeded in a 96 well plate at 1×10⁴ cells/well, and 25, 50 or 100 µL of the collected culture was taken to treat the cells, followed by confirmation of cancer cell killing ability of the viruses produced in the cell sheet by an MTT assay at 48 hours.

### Preparation of virus-loaded cell sheet

To prepare a cell sheet for replicating or delivering vaccinia virus or an adenovirus, a silicone ring having an inner diameter of 1.8 mm was placed on a temperature-responsive culture dish (TRCD), 3×10⁵ cells of an NIH3T3 cell line were seeded in the silicone ring, and after 48 hours, 3×10⁵ cells of a U343 cell line were seeded, thereby obtaining a cell sheet.

After 24 hours, the cell sheet was infected with vaccinia virus or an adenovirus. After a predetermined time, the culture medium of the cell sheet infected with the virus and the silicone ring were removed from the temperature-responsive culture dish and washed three times with 1× PBS, 2 mL of fresh 1× PBS was added, and then the cell sheet was detached at 20 °C to confirm the formation of a cell sheet.

### Experimental results

### Generation and characterization of oAd-DCN/CFCSs

To prepare CFCS which allows adenovirus replication, a double-layered cell sheet composed of a human brain glioblastoma cell line (U343) and a mouse fibroblast cell line (NIH3T3) was used.

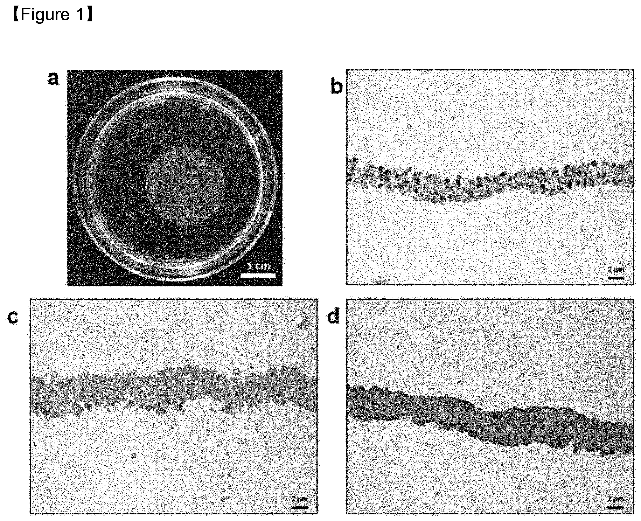

The CFCSs were generated in a temperature-responsive culture dish (TRCD), and infected with oAd-DCN at 5 MOI, thereby generating oncolytic adenovirus-infected CFCSs (oAd-DCN/CFCSs). At 12 hours after infection, the plate temperature was lowered to room temperature for 30 minutes, thereby separating oAd-DCN/CFCSs from the TRCD. As shown in FIG. 1A, a circular sheet with a uniform size was easily separated from the TRCD. H&E staining of the sheet showed that both cancer cell and normal fibroblast components were present in the sheet layer (FIG. 1B).

Importantly, Ad E1A staining of the cell sheet showed a broad distribution of oncolytic adenoviruses as seen in red spots (FIG. 1D). On the other hand, a PBS-treated control sheet had no detectable spots (FIG. 1C). As noted, PBS and oncolytic adenovirus-loaded CFCSs showed similar structures and shapes at 12 hours after infection. Taken together, these results demonstrate that oncolytic adenoviruses can be loaded in a cell sheet, which does not adversely affect overall structural integrity.

### Virus replication and gene expression profile within oAd-DCN/CFCSs

To assess whether the cancer cell layer of the sheet allows oncolytic adenovirus infection, viral replication and a therapeutic gene expression profile were analyzed by Q-PCR and western blotting, respectively.

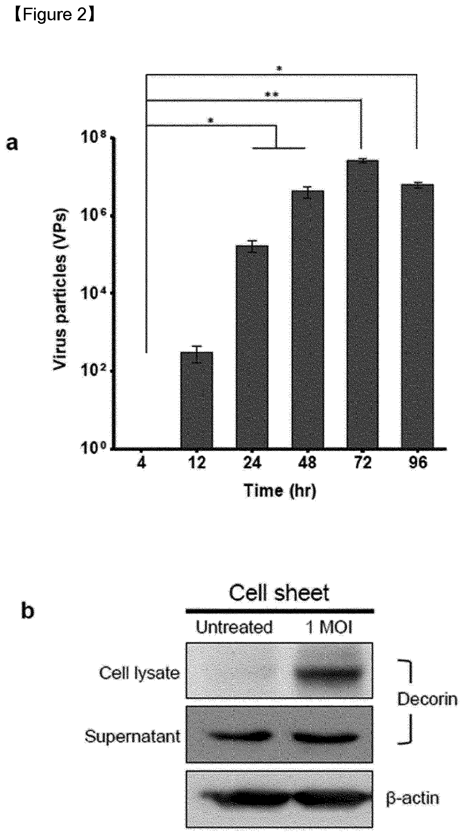

As shown in FIG. 2A, oncolytic adenoviruses were effectively replicated in a cell sheet over time up to 72 hours after infection, proving that the cell sheet allows oncolytic adenovirus replication (at 4 hours, a dose of viruses infected into the cell sheet was detected. However, due to a small MOI of viruses, Q-PCR data was below the detection limit and not analyzed).

At 96 hours after treatment, due to oncolytic activity of oAd-DCN, a slight decrease in Ad amount was observed, and at 96 hours after treatment, significant degradation of the cell sheet was induced. According to these results, western blot was performed and revealed that oAd-DCN can effectively generate DCN in oAd-DCN/CFCSs (FIG. 2B).

Taken together, these results demonstrated that the cell sheet is susceptible to oncolytic Ad infection, and ultimately serves as a delivery scaffold allowing viral replication and therapeutic gene expression.

### Degradation profile and viral persistence of oAd-DCN/CFCSs in vivo

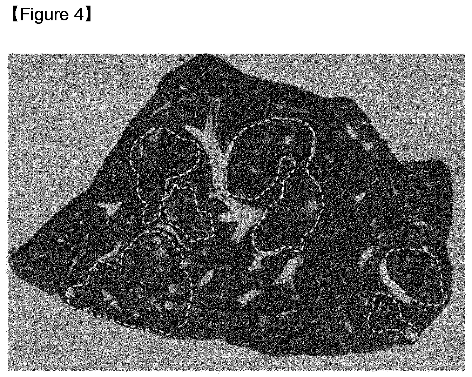

To monitor and visualize the degradation of the cell sheet and persistence of loaded oncolytic adenoviruses in CFCS, HCC xenograft mice were transplanted with oAd-DCN-loaded fluc-expressing CFCSs (oAd-DCN/CFCSs/Fluc) (FIG. 3A) or CFCSs (without a reporter gene) infected with fluc-expressing oAd-DCN (oAd-DCN-Fluc/CFCSs) (FIG. 4B).

As shown in FIG. 3A, the luciferase signal in the cell sheet was decreased in a time-dependent manner, and completely disappeared at 10 days after transplantation.

In addition, at 10 days after transplantation, mice were sacrificed, and then it was visually confirmed that all transplanted CFCSs were completely degraded in the liver.

Similarly, an oncolytic adenovirus signal was also decreased in a time-dependent manner, showing a similar time-dependent decrease in a luciferase expression pattern for 10 days, like the cell sheet (FIG. 3B).

### Potent antitumor efficacy of oAd-DCN/CFCSs against multifocal hepatocellular carcinoma

The orthotopic tumor model is emerging as one of the important cancer research models due to its clinical relevance. Since the multifocality of HCC is a critical challenge for successful therapy in clinic, a multifocal HCC orthotopic tumor model was established by multiple injection of the largest lobe of the liver with Hep3B cells expressing luciferase.

As shown in FIG. 4, HCC lesions were detected in the liver of PBS-treated mice, confirming that orthotopic multifocal HCC was successfully established.

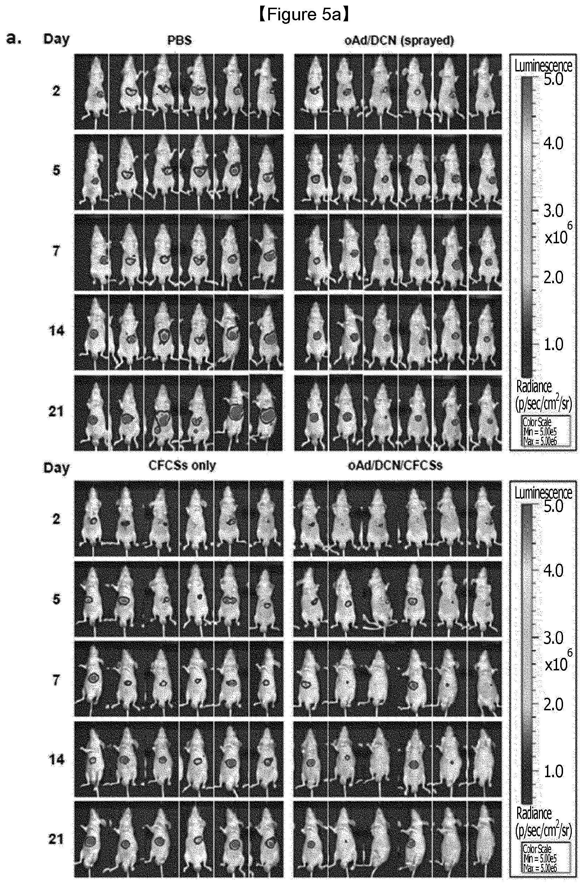

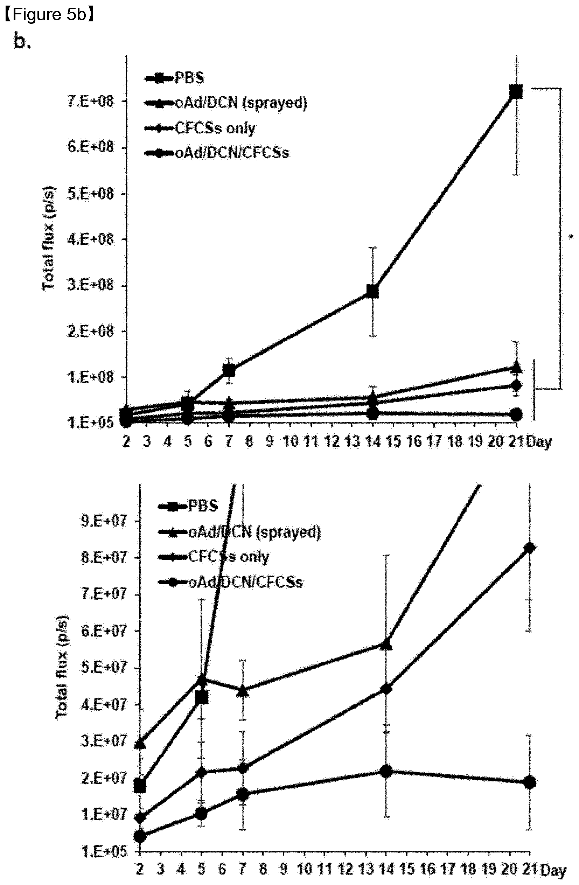

As shown in FIG. 5A, oAd-DCN/CFCS treatment showed significantly higher antitumor activity than any other treated groups at day 21 after treatment, and thus 20.4-, 4.7- or 3.5-fold greater inhibition of tumor growth was shown, compared to PBS (7.2×10⁸ ± 4.5×10⁸), PBS-treated CFCS (1.7×10⁸ ± 9.3×10⁷), or oAd-DCN (sprayed) (1.2×10⁸± 1.3×10⁸) (FIG. 5B).

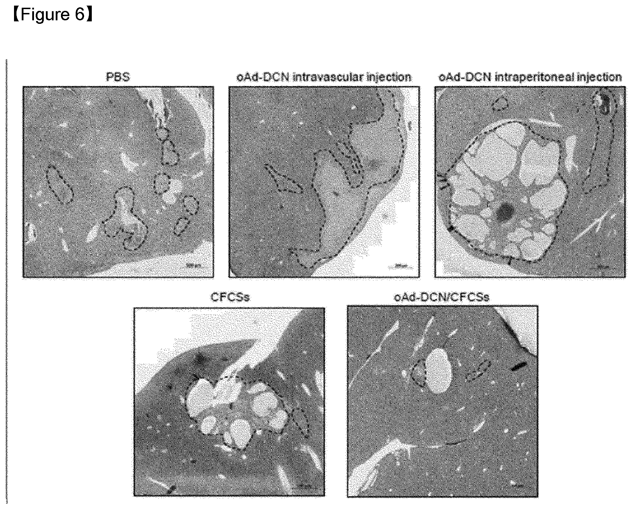

In addition, multifocal HCC was not observed in the livers of all oAd-DCN/CFCS-treated mice, suggesting that CFCS-mediated delivery of oncolytic adenoviruses can prevent formation of multifocal tumors (FIG. 6).

Taken together, these results suggest that oAd-DCN loaded in the cell sheet can be effectively delivered to tumor tissues to elicit potent antitumor efficacy and prevent the formation of multifocal HCC.

### Histological analyses of oAd-DCN/CFCSs against multifocal HCC

To further investigate a therapeutic effect, tumor tissues were harvested at 2 weeks after treatment with each group (PBS, oAd-DCN intravascular injection, oAd-DCN intraperitoneal injection, CFCS only or oAd-DCN/CFCSs), and then analyzed by histological and immunohistological analyses.

As shown in FIG. 6, H&E staining showed a large multifocal region of tumor cells proliferated in tissues or macrophages treated with PBS, intravascular injection of oAd-DCN, intraperitoneal injection of oAd-DCN or CFCS only.

In oAd-DCN/CFCSs, a small region of tumor cells and multifocal tumors were not observed. Taken together, it was proved that when CFCS is used for oAd local delivery, antitumor efficacy is enhanced, and the growth of multifocal tumors is prevented.

### Confirmation of biological activity and tumorigenesis after transplantation of cell sheet onto tumor-removed (target) region

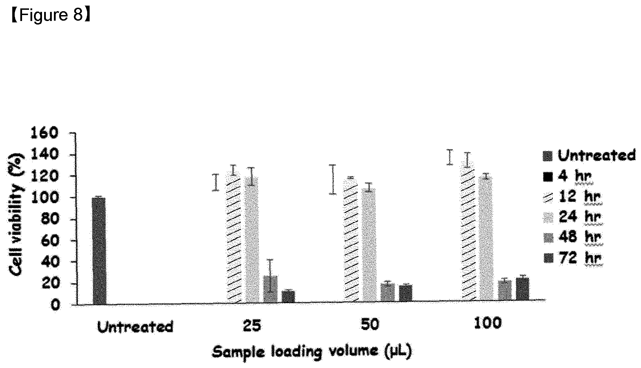

FIG. 8 shows the biological activity of an adenovirus replicated from an oncolytic adenovirus-loaded cell sheet, and by confirming that apoptosis increases in samples at late time points, which have larger amounts of virus replication, demonstrates biological activity of an adenovirus replicated from a cell sheet.

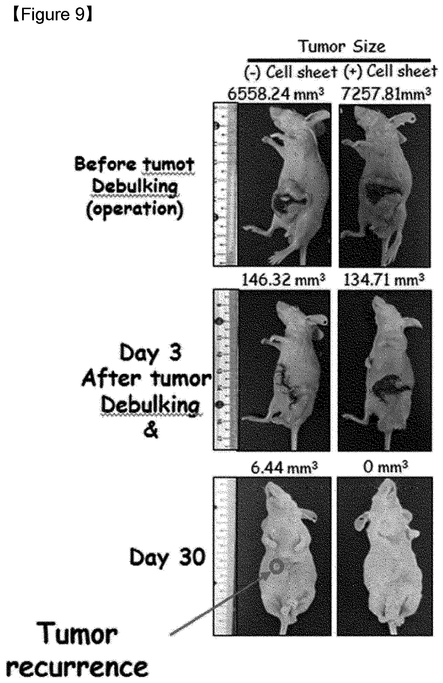

FIG. 9 shows the recurrence of tumors after an oncolytic adenovirus-loaded cell sheet is attached following tumor resection. After tumor resection, tumor recurrence was observed in the control to which the cell sheet is not attached 30 days after surgery, whereas tumors did not reoccur in the experimental group in which the cell sheet is attached to a tumor region and then sutured.

### Confirmation of vaccinia virus-infected cell sheet formation

It was confirmed that the cell sheet was well formed regardless of viral infection.

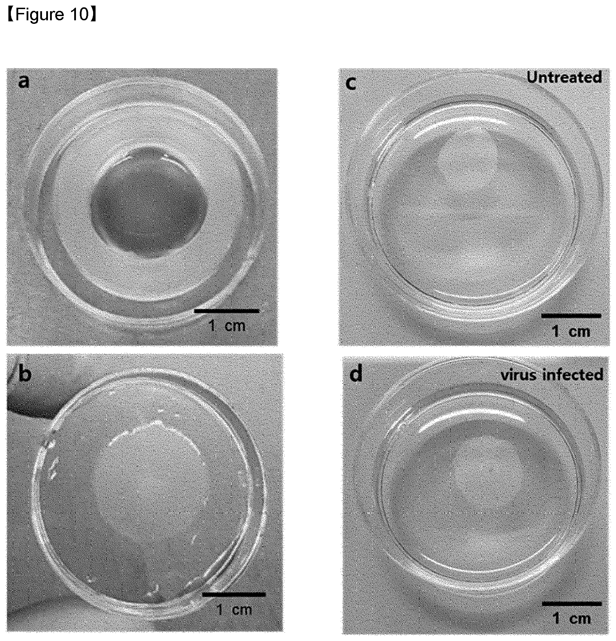

FIG. 10 shows (a) an image in which a cell sheet is infected with vaccinia viruses at 0.5 MOI in a temperature-responsive culture dish and then maintained for 6 hours, (b) an image in which a silicone ring is removed from the cell sheet and washed three times with 1X PBS in order to detach the cell sheet from the temperature-responsive culture dish, (c) an image in which after the temperature responsive culture dish is lowered at 20°C for 15 munutes the cell sheet is detached from the culture dish without viral infection, and (d) an image of the cell sheet infected with vaccinia viruses at 0.5 MOI.

### Confirmation of vaccinia virus replication ability in cell sheet

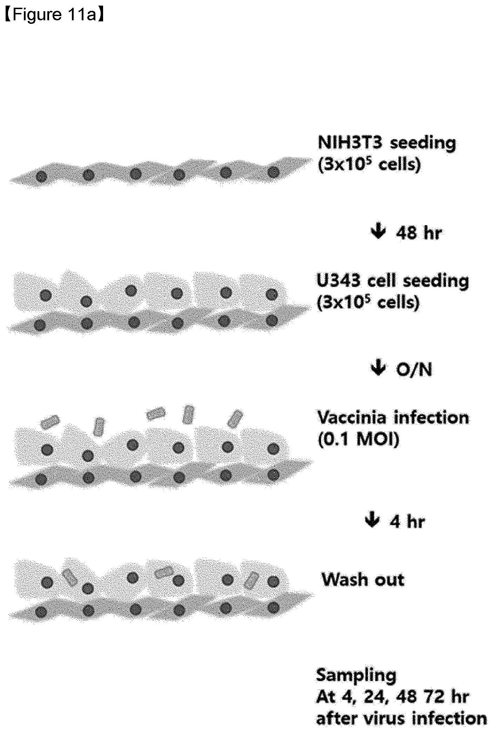

To assess the replication ability of vaccinia virus in a cancer cell layer of a cell sheet, as shown in FIG. 11A, a cell sheet composed of a first cell layer of fibroblasts and a second cell layer of cancer cells was formed, infected with vaccinia viruses at 0.1 MOI and washed to remove uninfected vaccinia viruses at 4 hours after infection, and then vaccinia viruses were detected from a cell culture solution and the cell sheet by Q-PCR at 24, 48 and 72 hours after infection.

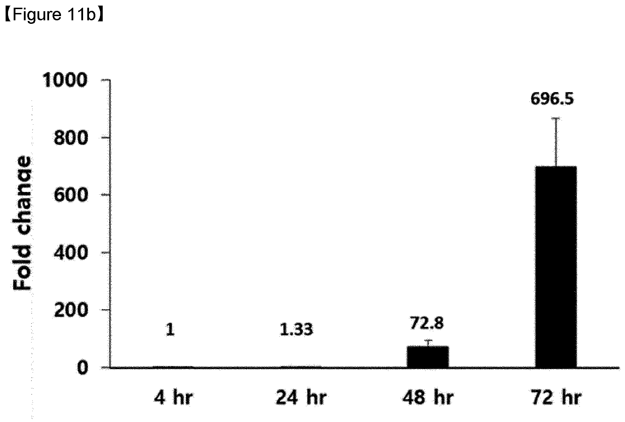

As shown in FIG. 11B, the viruses were effectively replicated in the cell sheet over time up to 72 hours after vaccinia virus infection, demonstrating that the cell sheet allows the replication of vaccinia viruses.

### Confirmation of apoptosis in cell sheet caused by vaccinia virus

To confirm apoptosis in cell sheet caused by viral infection, as shown in FIG. 11A, a cell sheet composed of a first cell layer of fibroblasts and a second cell layer of cancer cells was formed and infected with vaccinia viruses at 2 or 5 MOI, followed by addition of a 7-AAD dye which can stain dead cells in red to confirm apoptosis over time.

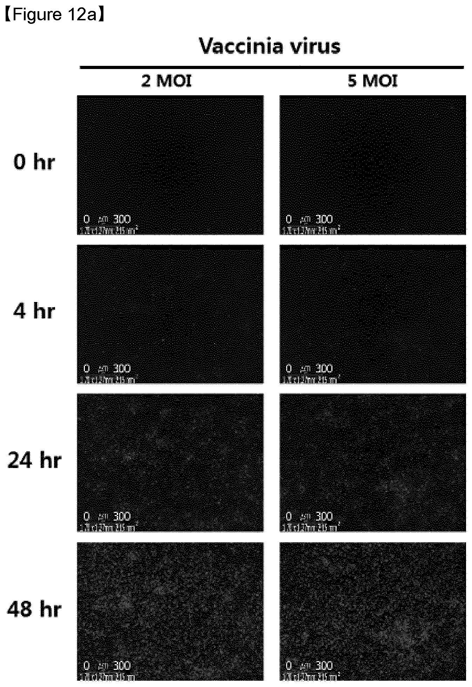

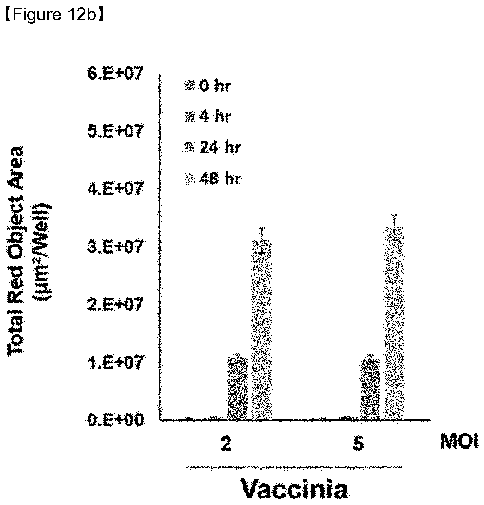

As shown in FIG. 12A, it was confirmed that the number of dead cells is increased in both of the cell sheets infected with vaccinia viruses at 2 and 5 MOI, and red areas in the images were quantified (FIG. 12B).

As combining the results of FIGS. 11 and 12, it was demonstrated that the cell sheet can be infected with vaccinia viruses, as well as adenoviruses, allows their replication, and thereby has biodegradability.

### Confirmation of cell viability of irradiated cancer cells

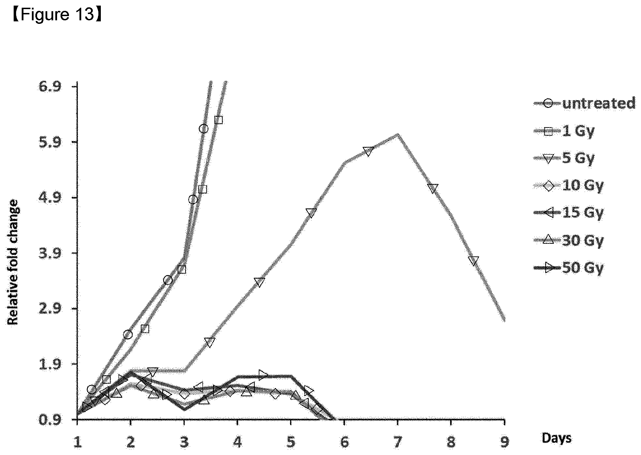

The cell viability of U343 cells which were irradiated with various intensities (1, 5, 10, 15, 30 and 50 Gy) of radiation was confirmed by an MTT assay. In the U343 cell line irradiated with 1 Gy, cells divided to a similar extent to that of non-irradiated cells (control), and when the cells were irradiated with 5 Gy, a cell division rate was lower than that of the control, and at 7 days after irradiation, the cells started to die. When U343 cells were irradiated with 10 to 50 Gy, there was very little cell division, and at 6 days after irradiation, all cells died (FIG. 13).

### Confirmation of Ad replication ability in cell sheet including irradiated cancer cells

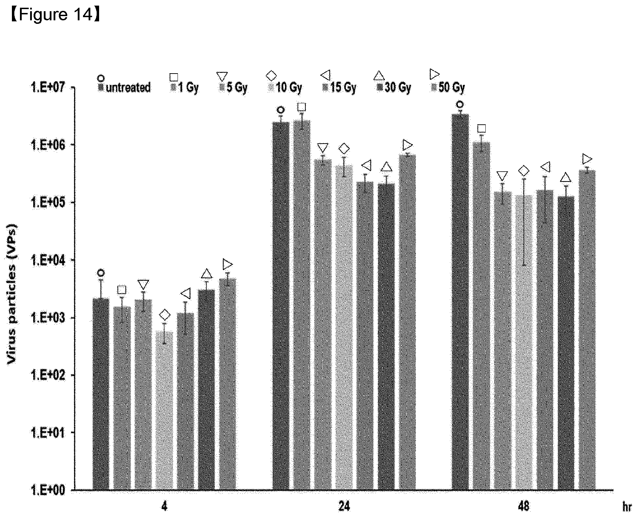

To assess the virus replication ability in an irradiated cancer cell layer of a cell sheet, as shown in FIG. 11A, a cell sheet composed of a first cell layer of fibroblasts and a second cell layer of cancer cells was formed, infected with adenoviruses at 0.5 MOI and washed to remove uninfected adenoviruses at 4 hours after infection, followed by confirmation of adenoviruses from a cell culture solution and the cell sheet by Q-PCR at 24, 48 and 72 hours after infection. As a result, it was confirmed that Ad replication occurs even in the irradiated cancer cells (FIG. 14).

### Discussion

Multifocal HCC often occurring due to chronic hepatic stress has many mutations and covers a large area of the liver, and therefore, it is difficult to perform curative resection in most patients.

Even patients receiving a resection show a poor prognosis of a 5-year survival rate of 50% due to a high recurrence rate.

In addition, since the extensive liver damage in these patients leads drugs to cause severe hepatotoxicity, such a chemotherapeutic agent may not be systemically administered at a proper dose.

To overcome the limitations of traditional HCC treatment options, a biodegradable and adenovirus replication-permissive cell sheet was generated to efficiently deliver oncolytic adenoviruses to multifocal loci of HCC.

The viral replication-permissive feature of a cell sheet delivery platform enables effectively treatment of multifocal HCC at a relatively low dose of viruses, compared with other conventional treatment routes (most local and systemic doses of oncolytic adenoviruses require 1-5 × 10¹⁰ VP).

At an equal virus dose, oAd-DCN/CFCS treatment leads to long-term release of oncolytic adenoviruses, showing excellent tumor growth inhibition and prevention of multifocal HCC formation, compared with systemic or local administration of naked oAd-DCN (FIG. 6).

One of the explanations for this is viral replication-mediated cell lysis and that a large region of a multifocal tumor region can be infected with most virions reaching the tumor, which are released from CFCS following.

Ultimately, maintenance of the infectivity of therapeutic viruses for a long time remains a principal hindrance in clinical trials. There are several other local delivery platforms, such as a hydrogel, a patch, and intratumoral injection which is currently under development to enhance localization and therapeutic efficacy of a therapeutic agent [Pesonen, S., L. Kangasniemi, and A. Hemminki, Oncolytic adenoviruses for the treatment of human cancer: focus on translational and clinical data. Mol Pharm, 2011. 8(1): p. 12-28; Wang, C., et al., Enhanced Cancer Immunotherapy by Microneedle Patch-Assisted Delivery of Anti-PD1 Antibody. Nano Lett, 2016. 16(4): p. 2334-40; Kasala, D., et al., Evolving lessons on nanomaterial-coated viral vectors for local and systemic gene therapy. Nanomedicine (Lond), 2016. 11(13): p. 1689-713]. One of the major problems of such platforms is that these platforms frequently use synthetic components such as polymers, liposomes and nanoparticles, and degradation products can cause inflammation and other side effects. However, since the oncolytic virus-loaded cell sheet of the present invention (e.g., oAd-DCN/CFCS), unlike other conventional local delivery platforms, includes no synthetic component, it is highly biocompatible and degradable. One of the critical concerns and hindrances for the CFCS approach in clinics is the use of a cancer cell layer supporting viral replication since it may generate other tumors. To address this concern, CFCS was generated using irradiated cancer cells to induce eradication of the cancer cell layer after initial transplantation, supporting viral replication (FIG. 13). In addition, irradiation enhanced viral replication with the cell sheet (FIG. 14).

These results show that the replication of oncolytic adenoviruses can be enhanced by DNA damage caused by irradiation and adjuvant radiation therapy, and the acceleration of DNA repair may result in greater replication of episomal adenovirus DNA.

Further, irradiated cancer cells are currently evaluated as a promising candidate for a cancer vaccine. This is because these cancer cells can provide tumor-associated antigens for a host immune system for recognizing and inducing a tumor-specific immune response, demonstrating a potential to become a promising immunotherapy platform for co-delivery of a tumor vaccine and an oncolytic adenovirus.

This potential immunological regulation of CFCS-mediated delivery of oncolytic adenoviruses is currently being evaluated as future research.

### Conclusion

In some embodiments of the present invention, a delivery system efficient for multifocal tumor treatment was made by the combination of oncolytic viruses and a cell sheet, and this system exhibited efficient proliferation and persistent release, and prevents non-specific release of oncolytic adenoviruses (oAd) due to a permissive cell sheet.

In conclusion, some embodiments of the present invention show that the use of an oncolytic virus/cell sheet system can maximize the therapeutic effect of oncolytic viruses by overcoming limitations of conventional cancer gene therapy.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect. Particularly, according to the embodiments described herein, the cell sheet is described as being used with the viruses described above, but the viruses can be replaced with other viral systems.

## Claims

1. A cell sheet comprising two or more cell layers, wherein a gene delivery system is introduced to one or more of the cell layers.

2. The cell sheet of claim 1, wherein the cell sheet includes a cell layer containing somatic cells as a support.

3. The cell sheet of claim 2, wherein the somatic cells are one or more selected from the group consisting of fibroblasts, chondrocytes, epithelial cells, myoepithelial cells, dermal cells, epithelial keratinocytes, Schwann cells, glial cells, osteoblasts, cardiomyocytes, megakaryocytes, adipocytes, stem cells and cancer cells.

4. The cell sheet of claim 1, wherein the gene delivery system is in the form of (i) a naked recombinant DNA molecule, (ii) a plasmid, (iii) a viral vector, and (iv) a liposome or niosome containing the naked recombinant DNA molecule or plasmid.

5. The cell sheet of claim 4, wherein the viral vector is one or more selected from the group consisting of a recombinant adenovirus, an adeno-associated virus (AAV), a retrovirus, a lentivirus, Herpes simplex virus, vaccinia virus, a reovirus, a poxvirus, Semliki forest virus and Measles virus.

6. The cell sheet of claim 1, wherein the gene delivery system is a recombinant adenovirus.

7. The cell sheet of claim 6, wherein the recombinant adenovirus is a replication-incompetent adenovirus or oncolytic adenovirus.

8. The cell sheet of claim 1, wherein the cell layer to which the gene delivery system is introduced includes cancer cells or stem cells.

9. The cell sheet of claim 1, comprising a cell layer containing somatic cells as a support; and a cell layer containing cancer cells or stem cells,
wherein a recombinant adenovirus is introduced to the cell layer containing cancer cells or stem cells.

10. The cell sheet of claim 1, wherein the cell sheet comprises a cell layer containing cancer cells, to which a gene delivery system is introduced,
wherein the cancer cells are irradiated.

11. The cell sheet of claim 4, wherein the viral vector is loaded at a multiplicity of infection (MOI) of 0.1 to 500.

12. A method of preparing a cell sheet, comprising:
forming a cell sheet including two or more cell layers on a temperature-responsive culture dish including a temperature-responsive polymer;
introducing a gene delivery system to one or more of the cell layers, and
separating the cell sheet from the temperature-responsive culture dish.

13. The method of claim 12, wherein the temperature-responsive polymer is one or more selected from the group consisting of poly(N-isopropylacrylamide), poly(N-vinylcaprolactame), polycaprolactone (PCL) and polylactate-co-glycolate (PLGA).

14. The method of claim 1, wherein the cell sheet includes a cell layer containing somatic cells as a support.

15. The method of claim 14, wherein the somatic cells are one or more selected from the group consisting of fibroblasts, chondrocytes, epithelial cells, myoepithelial cells, dermal cells, epithelial keratinocytes, Schwann cells, glial cells, osteoblasts, cardiomyocytes, megakaryocytes, adipocytes, stem cells and cancer cells.

16. The method of claim 12, wherein the gene delivery system is in the form of (i) a naked recombinant DNA molecule, (ii) a plasmid, (iii) a viral vector, and (iv) a liposome or niosome containing the naked recombinant DNA molecule or plasmid.

17. The method of claim 16, wherein the viral vector is one or more selected form the group consisting of a recombinant adenovirus, an adeno-associated virus (AAV), a retrovirus, a lentivirus, Herpes simplex virus, vaccinia virus, a reovirus, a poxvirus, Semliki forest virus and Measles virus.

18. The method of claim 12, wherein the gene delivery system is a recombinant adenovirus.

19. The method of claim 18, wherein the recombinant adenovirus is a replication-incompetent adenovirus or oncolytic adenovirus.

20. The method of claim 12, wherein the cell layer to which the gene delivery system is introduced includes cancer cells or stem cells.

21. The method of claim 12, comprising
forming a cell layer containing somatic cells as a support; forming a cell layer containing cancer cells or stem cells on the cell layer, and introducing a recombinant adenovirus to the cell layer having cancer cells or stem cells.

22. The method of claim 20 or 21, wherein the cancer cells are irradiated.

23. The method of claim 1, wherein the viral vector is inoculated at a viral dose of 0.1 to 500 multiplicity of infection (MOI).

24. A gene therapeutic agent comprising the cell sheet according to claim 1.

25. The gene therapeutic agent of claim 24, which is for prevention or treatment of cancer.

26. The gene therapeutic agent of claim 24, which is for prevention of cancer recurrence or metastasis.

27. The gene therapeutic agent of claim 25 or 26, wherein the cancer is one or more selected from the group consisting of multifocal hepatocellular carcinogenesis, glioma, glioblastoma, laryngeal cancer, pancreatic cancer, lung cancer, non-small cell lung cancer, colon cancer, bone cancer, pancreatic cancer, skin cancer, head and neck cancer, ovarian cancer, uterine cancer, rectal cancer, gastric cancer, anal cancer, colorectal cancer, breast cancer, fallopian cancer, endometrial cancer, cervical cancer, vaginal cancer, vulva cancer, Hodgkin's disease, esophageal cancer, small intestine cancer, endocrine gland tumors, thyroid cancer, parathyroid carcinoma, adrenal cancer, soft tissue sarcoma, urethral cancer, penile cancer, prostate cancer, chronic or acute leukemia, lymphocyte lymphoma, bladder cancer, kidney or urinary tract cancer, renal cell carcinoma, renal pelvic carcinoma, central nervous system (CNS) tumors, primary CNS lymphoma, spinal tumors, liver cancer, bronchial cancer, nasopharyngeal cancer, brainstem glioma and pituitary adenoma.

28. A method of preventing cancer recurrence, comprising transplanting the cell sheet of claim 1 onto a cancer-removed region.

29. A method of treating cancer, comprising transplanting the cell sheet of claim 1 onto a region in need of cancer treatment.
